(19) Europäisches Patentamt
European Patent Office
Office européen des brevets

(11) Veröffentlichungsnummer: **0 251 058**
**A2**

(12) # EUROPÄISCHE PATENTANMELDUNG

(21) Anmeldenummer: **87108778.9**

(51) Int. Cl.⁴: **C07B 55/00**

(22) Anmeldetag: **19.06.87**

Die Bezeichnung der Erfindung wurde geändert (Richtlinien für die Prüfung im EPA, A-III, 7.3).

(30) Priorität: **28.06.86 DE 3621835**

(43) Veröffentlichungstag der Anmeldung: **07.01.88 Patentblatt 88/01**

(84) Benannte Vertragsstaaten: **AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Wullbrandt, Dieter Dr.**
**Bienerstrasse 29**
**D-6238 Hofheim am Taunus(DE)**
Erfinder: **Schlingmann, Merten, Dr.**
**Schneidhainer Strasse 32a**
**D-6240 Königstein/Taunus(DE)**

(54) **Verfahren zur Racemisierung optisch aktiver- Phenoxy- propionsäureester und deren Derivate.**

(57) Optisch aktive Enantiomere der α-Phenoxypropionsäurealkylester und von deren Derivaten können mit Hilfe von Alkali($C_1$-$C_5$)Alkoholat, Alkalihydrochinon, Alkaliphenolat oder Alkali-Hydroxyphenoxypropionsäure sowie deren Derivaten in guten Ausbeuten und ohne Bildung von Zersetzungsprodukten racemisiert werden.

EP 0 251 058 A2

## Verfahren zur Racemisierung optisch aktiver α-Phenoxypropionsäure und deren Derivate

Viele, aus der Literatur bekannte, synthetische Wuchsstoffe und Herbizide oder deren Vorstufen, wie z.B. α-Phenoxypropionsäure, liegen bei ihrer Verwendung oder Weiterverarbeitung in Form von Racematen vor. Häufig ist jedoch eines der optisch aktiven Isomere wirksamer als das andere bzw. als das Racemat. Da es oft wirtschaftlicher ist, die entsprechende Substanz nur in der wirksameren Form einzusetzen, versucht man, die Racemate in ihre Enantiomere aufzutrennen. Um jedoch nicht mit einer 50 %igen Ausbeute zufrieden sein zu müssen, wird vielfach versucht, das inaktive Isomer zu racemisieren und das daraus resultierende Racemat wieder in den Kreislauf zurückzuführen. Auf diese Weise gelangt man zu einem kontinuierlichen Prozeß zur Herstellung der gewünschten optisch aktiven Verbindung.

Cambou und Klibanov beschrieben in einer Veröffentlichung (Biotechnol., Bioeng., Vol 26, 1449, 1984) die vollständige Racemisierung von L-Chlorphenoxypropionsäuremethylester durch Inkubation der in Methanol gelösten Substanz in Gegenwart katalytischer Mengen von Natrium-Methanolat bei 60°C über einen Zeitraum von 16 Stunden. In anderen Verfahren wird durch Erhitzen unter Verwendung eines Katalysators oder Lösemittels, wie z.B. die 2-Aminoalkyl oder Arylverbindungen (Deutsche Offenlegungsschrift 29 03 589 und Japanische Offenlegungsschrift 5 0050 317), oder durch Erhitzen der N-Acylaminosäuren mit Carbonsäuren (Deutsche Offenlegungsschrift 33 34 849A1) racemisiert.

Es hat sich jedoch herausgestellt, daß Derivate der α-Phenoxypropionsäure durch Erhitzen auf ca. 250°C in Substanz bzw. durch Zugabe von Hydrochinon zersetzt werden.

Es wurde nun überraschend gefunden, daß die Racemisierung der beiden stereoisomeren Formen der α-Phenoxypro pionsäure sowie von deren Derivaten mit Hilfe von Alkalialkoholaten im weiteren Sinne und Alkali-Hydroxyphenoxypropionsäure in guten Ausbeuten, ohne Bildung von Zersetzungsprodukten, durchgeführt werden kann.

Die Erfindung betrifft somit ein Verfahren zur Racemisierung optisch aktiver Enantiomere, das dadurch gekennzeichnet ist, daß die Verbindung der allgemeinen Formel I,

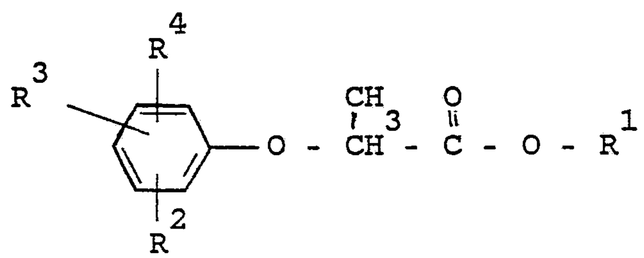

I

in der

a) $R^1$ eine Alkylgruppe mit bis zu 8 C-Atomen, die jeweils mit Hydroxy, Halogen, Alkyl und/oder Nitro substituiert sein können, oder eine cyclische Alkylgruppe mit bis zu 6 C-Atomen und

b) $R^2$ Wasserstoff, Hydroxyl oder eine Alkoxygruppe oder eine Aryloxygruppe oder ein über Sauerstoff gebundenes, heterozyklisches Ringsystem mit jeweils bis zu 10 C-Atomen, und

c) $R^3$ und $R^4$ unabhängig voneinander Wasserstoff oder Halogen, bedeuten, mit Hilfe von Alkali($C_1$-$C_5$)Alkoholat, Alkalihydrochinon, Alkaliphenolat und/oder Alkali-Hydroxyphenoxypropionsäure sowie deren Derivaten, die am aromatischen Ring Substituenten tragen, racemisiert wird.

Die Erfindung wird im folgenden detailliert beschrieben und in den Ansprüchen definiert.

Als Ausgangsprodukte dienen die Alkylester der α-Phenoxypropionsäure und von deren oben angeführten Derivaten. Die Alkylestergruppen $R^1$ können als Kette bis zu 8 Kohlenstoffatome, die substituiert sein können, bzw. als cyclische Al kylgruppe bis zu 6 C-Atome enthalten. Bevorzugt sind niedere gestreckte Alkylgruppen mit bis zu 5 C-Atomen, insbesondere unverzweigte Alkylgruppen mit 1 bis 3 C-Atomen.

Im folgenden erfindungsgemäßen Reaktionsverlauf wird der optisch aktive Ester in einem Lösemittel gelöst eingesetzt. Hierzu eignen sich beispielsweise inerte organische Lösemittel, in denen die L-und D-Ester löslich sind. Bevorzugt werden Kohlenwasserstoffe, wie Alkane mit einer Kettenlänge bis zu 10 Kohlenstoffatomen sowie Toluol oder Xylol, Dialkyl-Ether mit einer Kettenlänge bis zu 6 Kohlenstoffatomen, wie z.B. Di-isopropylether, Methylisobutylether, Methylisopropylether und tert. Butylmethylether, und Dialkyl-Ketone mit einer Kettenlänge bis zu 6 C-Atomen, wie z.B. Methylethylketon und Methylisobutylketon verwendet. Ferner werden Alkohole bevorzugt, wobei insbesondere der Alkohol eingesetzt wird, der zu der

Kettenlänge der Alkylgruppe $R^1$ der allgemeinen Formel I korrespondiert. Die Racemisierung wird, wie oben schon erwähnt, durch Zugabe von Alkali-($C_1$-$C_5$)Alkoholat, bevorzugt Alkali-($C_2$-$C_4$)Alkoholat, Alkalihydrochinon, Alkaliphenolat oder Alkali-Hydroxyphenoxypropionsäure durchgeführt. Es können auch Derivate der genannten aromatischen Verbindungen verwendet werden, insbesondere solche, die am aromatischen Ring bis zu zwei zusätzliche Substituenten tragen, die unabhängig voneinander Wasserstoff, Halogen, Alkyl und Alkoxy sind.

Die Reagenzien werden in Konzentrationen von 1 bis 10 Molprozent, bevorzugt 3 bis 8 Molprozent, eingesetzt. Wenn der α-Phenoxypropionsäureester oder dessen Derivate mit Alkali ($C_1$-$C_5$) Alkoholaten racemisiert werden, müssen mehr als 1,0 mol Base pro mol Ester eingesetzt werden. Unter Verwendung der übrigen genannten Reagenzien werden katalytische Mengen benötigt. Man erhitzt auf 100 bis 200°C und inkubiert 30 Minuten bis 8 Stunden, bevorzugt 2 bis 4 Stunden.

Wenn mit Alkali($C_1$-$C_5$)-Alkoholat racemisiert wird, erhält man in alkalischem Millieu weitgehend quantitativ die Säure als Endprodukt bzw. in saurem Millieu den Ester.

Die Abtrennung der Endprodukte aus dem Reaktionsgemisch erfolgt nach an sich bekannten Methoden, im Falle der Säure durch Ansäuern und Extraktion mit einem relativ unpolaren Lösungsmittel, wie beispielsweise Diethylether, oder im Falle des Esters beispielsweise durch Destillation.

Anhand von Beispielen wird die Erfindung weiter näher erläutert. Prozentangaben beziehen sich auf das Gewicht, sofern nicht anders angegeben.

## Beispiel 1

10 g L-2-(4-Hydroxyphenoxy)propionsäureethylester wurden in einer Lösung aus 50 ml tert. Butylmethylether und 22 ml of 30 %iger Natriummethylat-Lösung 2 Stunden am Rückfluß gehalten. Nach dem Abkühlen wurde das Reaktionsgemisch mit 50 ml Wasser versetzt, die wäßrige Phase abgetrennt und mit Salzsäure auf einen pH-Wert von 2,0 eingestellt. Die D,L-Säure wurde mit 50 ml Diethylether extrahiert. Nach dem Trocknen der organischen Phase mit Natriumsulfat wurde auf 10 ml eingeengt und zur Kristallisation der racemischen Säure mit 10 - 20 ml n-Hexan versetzt.
Ausbeute: 6,8 g D,L-2-(4-Hydroxyphenoxy)propionsäure

## Beispiel 2

10 g L-2-(4-Hydroxyphenoxy)propionsäureethylester wurden in einer Lösung aus 50 ml tert. Butylmethylether und 12 ml 30 %iger Natriumethylat-Lösung 2 Stunden auf Siedetemperatur erhitzt. Nach dem Abkühlen wurde das Reaktionsgemisch mit 5 ml Eisessig und anschließend mit 20 ml Wasser versetzt. Die organische Phase wird abgetrennt, getrocknet und das Lösungsmittel im Vakuum entfernt.
Ausbeute: 7,9 g D,L-2-(4-Hydroxyphenoxy)propionsäureethylester.

## Beispiel 3

100 mg Hydrochinon wurden durch Zugabe von 9 ml 0,1 molarer ethanolischer Kalilauge in das Monokaliumsalz übergeführt. Ethanol wurde im Vakuum entfernt und zu dem Rückstand 3 g L-2-(4-Hydroxyphenoxy)propionsäureethylester ($[\alpha]^{20}_{a}$ -41° (c = 2, in Chloroform)) gegeben. Anschließend wurde 3 h bei 170°C unter Luftaussschluß gerührt. Nach dem Abkühlen des Reaktionsgemisches wird der racemische Hydroxyphenoxypropionsäureethylester destillativ bei 130°C und 0,1 Torr abgetrennt.
Ausbeute: 2,4 g D,L-2-(4-Hydroxyphenoxy)propionsäureethylester (80 % der Theorie).

## Beispiel 4

Man verfährt analog Beispiel 3 unter Zugabe von 100 mg Natriumphenolat.
Nach dem Aufarbeiten wurden 2,5 g racemischer D,L-2-(4-Hydroxyphenoxy)propionsäureethylester erhalten.

Beispiel 5

0,3 g 2-(4-Hydroxyphenoxy)propionsäure in Form des Natriumsalzes wurden zu 3 g L-2-(4-Hydroxyphenoxy)propionsäureethylester gegeben. Man erwärmte die Reaktionsmischung unter Luftausschluß auf 160, 180 und 200°C. Nach der Aufarbeitung gemäß Beispiel 3 wurde die Racemisierung mit Hilfe des spezifischen Drehwerts in Chloroform bestimmt.

| Zeit [h] | Temperatur [°C] | spez. Drehwert $[\alpha]_D^{20}$ |
|---|---|---|
| 3 | 160 | -33,0° |
| 3 | 180 | -24,2° |
| 3 | 200 | -3,2° |
| 1 | 200 | -9,7° |

**Ansprüche**

1. Verfahren zur Racemisierung optisch aktiver Enantiomere, dadurch gekennzeichnet, daß die Verbindung der allgemeinen Formel I,

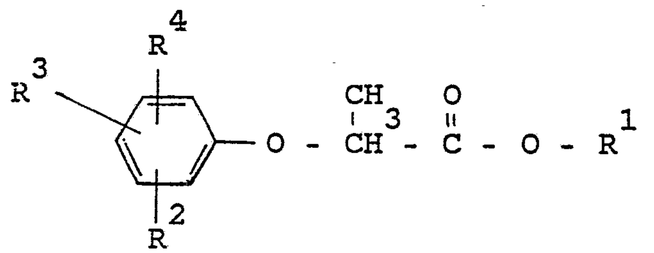

I

in der
a) $R^1$ eine Alkylgruppe mit bis zu 8 C-Atomen, die jeweils mit Hydroxy, Halogen, Alkyl und/oder Nitro substituiert sein können, oder eine cyclische Alkylgruppe mit bis zu 6 C-Atomen und
b) $R^2$ Wasserstoff, Hydroxyl oder eine Alkoxygruppe oder eine Aryloxygruppe oder ein über Sauerstoff gebundenes, heterozyklisches Ringsystem mit jeweils bis zu 10 C-Atomen, und
c) $R^3$ und $R^4$ unabhängig voneinander Wasserstoff oder Halogen,
bedeuten, mit Hilfe von Alkali($C_1$-$C_5$)Alkoholat, Alkalihydrochinon, Alkaliphenolat und/oder Alkali-Hydroxyphenoxypropionsäure sowie deren Derivaten, die am aromatischen Ring Substituenten tragen, racemisiert wird.

2. Verfahren nach Anspruch 1, dadurch gekennzeichnet, daß Alkalihydrochinon, Alkaliphenolate und/oder Alkali-Hydroxyphenoxypropionsäure eingesetzt werden, die bis zu zwei zusätzliche Substituenten am aromatischen Ring tragen, die unabhängig voneinander Sauerstoff, Halogen, Alkyl oder Alkoxy sind.

3. Verfahren nach Anspruch 1 oder 2, dadurch gekennzeichnet, daß die Reaktion in einem inerten organischen Lösungsmittel durchgeführt wird.

4. Verfahren nach Anspruch 3, dadurch gekennzeichnet, daß als Lösungsmittel Alkane mit einer Kettenlänge bis zu 10 Kohlenstoffatomen oder Toluol oder Xylol oder Dialkylether mit einer Kettenlänge bis zu 6 Kohlenstoffatomen oder Dialkylketone mit einer Kettenlänge bis zu 6 Kohlenstoffatomen oder der in der Kettenlänge zur Alkylgruppe $R^1$ der Ester der allgemeinen Formel I korrespondierende Alkohol eingesetzt werden.

5. Verfahren nach einem oder mehreren der Ansprüche 1 bis 4, dadurch gekennzeichnet, daß die Racemisierung bei 100 bis 200°C über einen Zeitraum von 30 Minuten bis 8 Stunden durchgeführt wird.

6. Verfahren nach Anspruch 5, dadurch gekennzeichnet, daß die Racemisierung über einen Zeitraum von 2 bis 4 Stunden verläuft.

7. Verfahren nach einem oder mehreren der Ansprüche 1 bis 6, dadurch gekennzeichnet, daß die Reagenzien in einer Konzentration von 1 bis 10 Molprozent eingesetzt werden.

8. Verfahren nach Anspruch 7, dadurch gekennzeichnet, daß die Reagenzien in einer Konzentration von 3-8 Molprozent eingesetzt werden.